# EUROPÄISCHE PATENTSCHRIFT

(11) **EP 0 543 868 B1**
(45) Veröffentlichungstag und Bekanntmachung des Hinweises auf die Patenterteilung: **30.11.1994**
(21) Anmeldenummer: 91914465.9
(22) Anmeldetag: 15.08.1991
(51) Int. Cl.: A61L 15/28, A61L 15/44, A61L 15/32

(54) **WUNDAUFLAGENSYSTEM**
DRESSING SYSTEM
SYSTEME DE PANSEMENT

(30) Priorität: 17.08.1990 DE 4026153
(43) Veröffentlichungstag der Anmeldung: 02.06.1993
(73) Patentinhaber: SEBA-PHARMA GMBH & CO., D-56136 Boppard (DE)
(72) Erfinder: RADEMACHER, Karl-Heinz, D-3000 Hannover 71 (DE); FRITSCHE, Ulrich, D-3000 Hannover 61 (DE)
(74) Vertreter: Werner, Hans-Karsten, Dr.Dipl.-Chem.
(86) Internationale Anmeldenummer: EP9101551
(87) Internationale Veröffentlichungsnummer: WO9203172

(56) Entgegenhaltungen:
- EP-A- 0 041 934
- EP-A- 0 047 796
- EP-A- 0 072 251
- WO-A-83/01384
- WO-A-84/01108
- DE-C- 3 045 225

## Beschreibung

Gegenstand der vorliegenden Erfindung ist ein Wundauflagensystem in Form eines Wundverbandes, einer Wundauflage oder einer Trägermatrix, bestehend aus einem bioverträglichen, offenporigen Kunststoffschaum, in dessen Poren ein Hydrogel eingelagert ist. Diese Strukturen können auch als bioverträgliche Matrix angesehen werden, in deren Netzwerk das Hydrogel eingelagert ist.

Ein Wundverband, der aus einem Netzwerk von Polyacrylamid einerseits und Agar andererseits besteht, wird unter der Bezeichnung Geliperm^{(R)} mit Erfolg eingesetzt, vergl. Biomaterials 1986, Volume 7, Seiten 67 bis 72. Okklusive Wundverbände der verschiedensten Art sind weiterhin beschrieben worden in dem Übersichtsartikel von Vincent Falanga in Arch. Dermatol, Volume 124, 1988, Seiten 872 bis 876. Der Artikel schließt mit der Erwartung, daß in der nahen Zukunft wohl mit weiteren aufsehenerregenden Entwicklungen auf dem Gebiet der okklusiven Therapie von Wunden zu rechnen ist. Tatsächlich ist festzustellen, daß für die Wundabdeckung chronisch nicht heilender Wunden, Ulcera, Decubitus, Brandwunden und schwerer Hautverletzungen, noch immer nicht der ideale und nahezu generell einsetzbare Wundverband entwickelt worden ist. Dies liegt insbesondere daran, daß die Heilung einer Wunde aus einer Wundheilungskaskade besteht, die sich aus ineinander übergehenden Phasen zusammensetzt, nämlich der Exsudation, der Granulation, der Epithelisation und schließlich der Marixbildung, die zur Wundkontraktion führt. Jede dieser Wundheilungsphasen ist mit einer Vielzahl biochemischer Reaktionen verbunden, die darauf ausgerichtet sind, schnellstmöglich die Wunde von Detritus und körperfremden Stoffen zu säubern und zu schließen.

Die bisher entwickelten Wundabdeckungen dienen vor allem dazu, die offene Wunde vor exogenen Einflüssen, wie Infektionen oder mechanischen Reizen jedweder Art zu schützen und ein Austrocknen der Wundoberfläche sowie einen Wärmeverlust zu verhindern.

Neueren Wundauflagen ist gemeinsam die absorbierende Eigenschaft der verwendeten Materialien. Sie sind meist in der Lage, Flüssigkeit aufzunehmen. Aus der DE-C-30 45 225 ist sogar ein besonders saugfähiges Material bekannt, welches aus entwässerten cis-1,2-Diol-polysacchariden besteht, die mit Borationen vernetzt und anschließend entwässert werden. Diese Produkte dienen vor allem zur Herstellung von stark Urin aufnehmenden Windeln. Sie können aber auch in einen Polyurethanschaum eingebracht werden und als stark Blut aufsaugendes Verbandsmaterial verwendet werden. Andere Wundverbände bestehen aus einem Film, welcher in der Lage ist, Wasserdampf, Sauerstoff und Kohlendioxid durchzulassen. Sie werden meist direkt auf die Wunde aufgebracht und sind in den ersten Phasen sehr wirksam. Bei beginnender Epithelisation jedoch führen sie dazu, neu gebildete Haut mitzureißen, sobald sie von der Wunde entfernt werden.

Sofern wasserundurchlässige Polyurethanschichten mit einem Hydrokolloid kombiniert werden, führt dies dazu, daß die Gas- und Wasserdampfdurchlässigkeit des Wundverbands vermindert ist, was sich meist durch die Bildung und den Austritt sehr unangenehm riechender gelbbrauner Flüssigkeiten bemerkbar macht; vgl. Vincent Falanga, loc. cit. Seite 875 "Hydrocolloids".

Die EP-A-0 041 934 beschreibt Polyurethanschäume, deren Hohlräume mit wasserunlöslichen, quellbaren Polysacchariden gefüllt sind. Letztere können Desinfektionsmittel und andere pharmakologisch aktive Substanzen enthalten.

Die WO-A-8 401 108 erwähnt unter anderem Polysaccahridträger, die mit Proteinen oder Proteinfragmenten beladen sind, die an dem Träger mittels CDI oder Bromcyan gekoppelt sind und dazu dienen, Mikroorganismen von verschiedenen Gewebearten zu entfernen.

Die WO-A-8 301 384 beschreibt eine zweischichtige Membran bestehend aus Silikon-elastomer/Kollagen, Glucosaminoglykan, wobei auf der Oberfläche der zweischichtigen Membran autolog gezüchtete Zellverbände aufgebracht sind, die der Heilung großflächiger Hautverletzungen dienen.

Verschiedene bisher bekannte Typen von Wundverbänden sind beispielsweise beschrieben in der EP-B-0 171 268, DE-A-32 24 382 und EP-A-0 335 669.

Weiterhin ist zur Wundbehandlung und Wundreinigung bereits von lytischen Enzymen aus der Gruppe der Proteasen Gebrauch gemacht worden, die als Lösung, Puder oder an Träger immobilisiert zur Anwendung gekommen sind. Beispiele hierfür finden sich in der DE-A-34 35 718, DE-A-34 44 746, DE-A-35 00 755 und DE-A-36 06 265. Die Behandlung mit diesen Enzymen ist meist mit Schmerzen verbunden. Außerdem werden gelegentlich allergische Reaktionen beobachtet aufgrund der in den Körper unkontrolliert eindiffundierenden Enzyme. Auch bei den an Mikroperlen gebundenen Enzymen besteht der Nachteil, daß Fremdstoffe in die Wunde eingetragen werden, die nach einer gewissen Einwirkungszeit vollständig aus der Wunde entfernt werden müssen, um Störungen in der endgültigen Wundheilung zu vermeiden. Dies wird mehr oder weniger vollständig durch Ausspülen des Wundgebietes mit geeigneten Lösungen erreicht. Dies wiederum führt meist zu einer Störung oder Unterbrechung des Heilungsprozesses.

Die vorliegende Erfindung hat sich die Aufgabe gestellt, einen Wundverband, eine Wundauflage oder eine Trägermatrix zur Verfügung zu stellen, welche die Vorteile der verschiedensten bekannten Wundverbände und Wundreinigungsmittel miteinander verbinden und gleichzeitig die Nachteile der verschiedensten bekannten Mittel vermeiden.

Der Wundverband soll somit in der Lage sein, Exsudat aufzusaugen, Gase, wie Sauerstoff, Kohlendioxid und Wasserdampf, durchzulassen, ein Austrocknen und Unterkühlen zu vermeiden, Detritus und körperfremde Stoffe zu entfernen, Infektionen von außen zu unterbinden, keine Allergien auszulösen und einen Wechsel des Wundverbandes zu ermöglichen, ohne bereits gebildetes Granulationsgewebe wieder zu zerstören. Keinesfalls dürfen Rückstände in der Wunde verbleiben, die die Wundheilung stören oder später zu Störungen führen.

Diese Aufgabe kann überraschend einfach gelöst werden durch einen Wundverband, eine Wundauflage oder eine Trägermatrix, bestehend aus einem bioverträglichen, offenporigen Kunststoffschaum, in dessen Poren eingelagert ist ein Hydrogel, welches gebildet ist aus mit Borat modifiziertem Guar gum und an welches zumindest oberflächlich gebunden sind über freie Hydroxyl- und/oder Aminogruppen und bifunktionelle Kopplungsreagenzien, die Wundheilung fördernde Peptide, vorzugsweise proteolytische Enzyme. Vorzugsweise ist zusätzlich die der Wunde abgewendete Seite abgedeckt mit einer wasserundurchlässigen, aber gas- und wasserdampfdurchlässigen Schicht. Diese Schicht ist vorzugsweise selbstklebend ausgebildet, kann jedoch auch in Form einer zweiten locker aufliegenden Schicht auf die Wunde aufgetragen werden.

Gewünschtenfalls ist es sogar möglich, in das Hydrogel weitere Wirkstoffe und/oder die Wundheilung fördernde Peptide einzulagern, welche verzögert und somit richtig dosiert an die Wunde abgegeben werden können.

Schließlich ist es möglich, auf der Oberfläche über Zelladhäsionsmoleküle autolog gezüchtete Zellverbände aufzubringen, welche der Heilung großflächiger Hautverletzungen dienen. Gegebenenfalls können diese Zellen auch gentechnologisch verändert sein, um dann mit ihrer Hilfe stoffwechselbedingte Wundheilungsstörungen zu behandeln.

Es ist somit erfindungsgemäß möglich, auf Basis des vernetzten Polysaccharid-Hydrogels modulartig Wundverbände, Wundauflagen oder eine Trägermatrix aufzubauen, welche die verschiedensten Funktionen für den Wundheilprozeß erfüllen können.

Als bioverträglich offenporiger Kunststoffschaum kommt Polyurethanschaum, insbesondere retikulierter Polyurethanschaum, in Frage, jedoch können prinzipiell auch andere bioverträgliche offenporige Kunststoffschäume verwendet werden, sofern es möglich ist, in sie Guar gum einzulagern und mit Borationen zu vernetzen. Dies kann gegebenenfalls auch dadurch geschehen, daß man das Guar gum mit anderen gelbildenden Substanzen, wie Agar, vermischt und dann mit Borationen vernetzt. Dabei entstehen miteinander verwobene offene Strukturen, die dazu führen, daß das Hydrogel durch das Netzwerk mechanisch stabilisiert wird, insbesondere auch dann, wenn es nach dem Auflegen auf die Wunde Flüssigkeit aufsaugt und dabei seine physikalische Konsistenz und Stabilität verändert. Vorzugsweise wird aber vorgefertigter, offenporiger Kunststoffschaum, beispielsweise Polyurethanschaum, verwendet, der einseitig mit einer Lösung von Guar gum mit breiiger Konsistenz beschichtet und danach durch Borat-Ionen verfestigt wird. Die Peptide werden entweder schon vorher oder nachträglich an das Guar gum-Gel gekoppelt.

Als Hydrogel wird erfindungsgemäß mit Borat modifiziertes Guar gum verwendet. Guar gum ist ein Peptidomannan, welches aus einer viskosen wäßrigen Lösung durch Zugabe einer Boratlösung in ein mechanisch stabiles Hydrogel überführt werden kann. Guar gum ist ein gut untersuchter, nichttoxischer Naturstoff, der wegen seiner besonderen metabolischen Eigenschaften auf den Kohlenhydrat- und Lipidstoffwechsel bei nicht Insulin abhängigem Diabetes mellitus als Therapeutikum eingesetzt wurde, vergl. Am. J. Clin. Nutr., 41 (5), Seiten 891 bis 894, 1985. Borat führt zur Bildung eines stabilen, leicht handzuhabenden Hydrogels, welches ausgesprochen gut bioverträglich ist.

Aufgrund der freien Hydroxylgruppen und Aminogruppen des Peptidomannans ist es möglich, an dieses Hydrogel mit Hilfe bifunktioneller Kopplungsreagenzien wundheilungsfördernde Peptide, insbesondere proteolytische Enzyme kovalent zu binden. Typische Kopplungsreagenzien sind Diisocyanate, Bromcyan, Glutaraldehyd und Carbodiimidazol.

Es ist ebenso möglich, wachstumsfördernde Substanzen, die die Wundheilung dosisabhängig beschleunigen, an die Oberfläche zu binden. Als Beispiel sind zellbindende Moleküle (CAM, Cell Adhesion Molecules) zu nennen.

Mit den zellbindenden Oberflächeneigenschaften des modifizierten Hydrogels sind folgende Funktionen verbunden: eine lokale Immunstimulation im Wundgebiet durch Bindung von Zellen und die Bindung von autolog gewonnenen Zellen und weitere Anzucht zu Zellverbänden.

Als proteolytische Enzyme kommen insbesondere in Frage Trypsin, Chymotrypsin, Kollagenase, Gelatinase, DNA'se sowie die Handelsprodukte Varidase^{(R)} und Trypure^{(R)}.

Da diese trägergebundenen proteolytischen Enzyme nur an der Oberfläche ihre Wirksamkeit zu entfalten brauchen, genügt es erfindungsgemäß, in den Kunststoffschaum CDI-aktiviertes Hydrogel einzubringen und danach mit der proteolytischen Enzymlösung zu besprühen. Überschüssige Reste an Kopplungsreagenz und/oder proteolytischem Enzym können durch Auswaschen und/oder Umsetzung mit Aminosäurelösungen oder wirkungsneutralen Peptiden abgesättigt werden. Die Kopplungsreaktion kann aber auch zunächst mit dem Polysaccharid durchgeführt werden, welches dann mit dem immobilisierten Peptid in eine Matrix eingelagert wird.

Insbesondere wenn die der Wunde abgewendete Seite abgedeckt ist mit einer wasserundurchlässigen, aber gas- und wasserdampfdurchlässigen selbstklebenden Schicht, kann die Umsetzung mit dem bifunktionellen Kopplungsreagenz und dem proteolytischen Enzym beschränkt werden auf die der Wunde zugewendeten Seite. Es ist produktionstechnisch aber ebenso gut möglich, den Kunststoffschaum mit eingelagertem Hydrogel beidseitig mit Kopplungsreagenz und Enzym zu behandeln, abzusättigen, auszuwaschen, zu trocknen und dann mit der selbstklebenden Schicht zu versehen.

Als gas- und wasserdampfdurchlässige Schicht kommt insbesondere Polyetherurethan in Frage. Da ein direkter Kontakt mit der Wunde nicht erforderlich ist, können aber auch andere Folien mit entsprechenden Eigenschaften zur Anwendung kommen, inklusive wasserundurchlässige, aber wasserdampf- und gasdurchlässige Gewebe, wie Gore-Tex^{(R)}.

Sofern die selbstklebende Schicht hautverträglich ist, kann diese Abdeckschicht größer dimensioniert werden als der Wundverband aus dem offenporigen Kunststoffschaum mit eingelagertem Hydrogel, so daß der Wundverband, die Wundauflage oder die Trägermatrix insgesamt mit Hilfe dieser selbstklebenden Schicht auf der Haut befestigt werden kann. Es ist aber ebenso gut möglich, den Wundverband, die Wundauflage oder die Trägermatrix einschließlich Abdeckfolie in größeren Stücken und Rollen herzustellen, bei Bedarf auf die notwendige Größe der Wunde zurechtzuschneiden und entweder mit einer Binde oder einem selbstklebenden Pflaster am Körper zu befestigen.

Wie bereits weiter oben erwähnt, können außer proteolytischen Enzymen auch andere die Wundheilung fördernde Peptide, wie Wundheilungsfaktoren gekoppelt werden. Auf diese Weise ist es möglich, in der Anfangsphase überwiegend oder nur proteolytische Enzyme zur Anwendung zu bringen und in späteren Phasen die proteolytischen Enzyme durch granulation- und epithelisationfördernde Faktoren zu ersetzen oder zu ergänzen. Schließlich ist es möglich, in das Hydrogel derartige Wirkstoffe und Faktoren einzulagern, da sie aus dem Hydrogel nur sehr langsam herausdiffundieren und in die Wunde gelangen. Geeignete Peptide sind beispielsweise die den Heilungs- und Regenerationsprozeß steuernden Glycoproteide wie Fibrinogen, Fibronectin, Fibrinolysin, Plasmin, Streptokinasen, Gerinnungsfaktoren etc., von Thrombozyten abgeleiteter Wachstumsfaktor, Heparin bindender Wachstumsfaktor, epidermaler Wachstumsfaktor, Granulozyten stimulierender Wachstumsfaktor, Makrophagen stimulierender Wachstumsfaktor, Ascorbat, Tretinoin, Hormone, Mediatoren, Matrixproteine und aktive synthetische Peptidsequenzen. Als weitere Wirkstoffe kommen vor allem Antibiotika, wie Chloramphenicol, Bacitracin, Polymyxin, Tetracycline, Penicilline, Cephalosporine etc., in Frage.

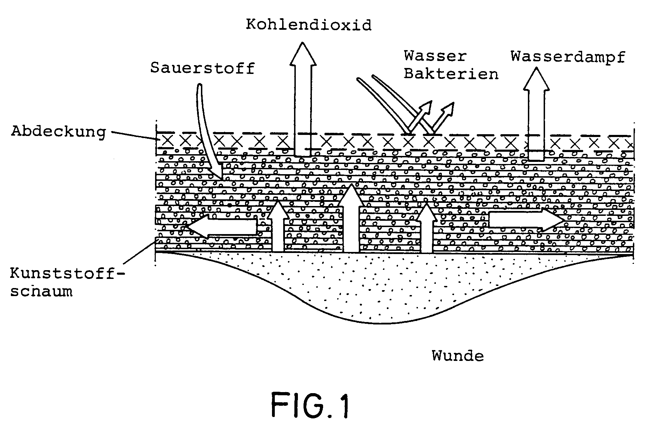

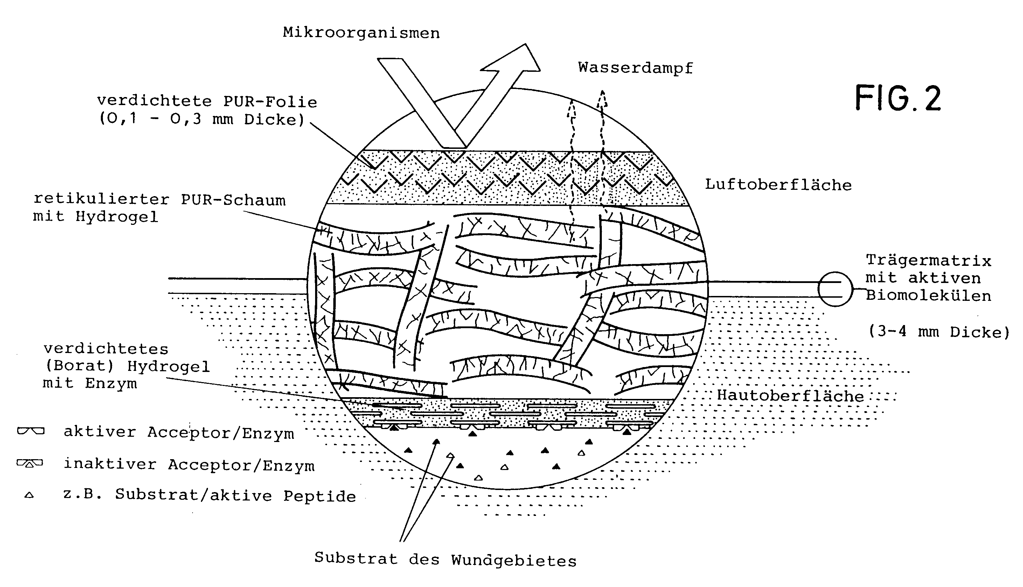

Der Aufbau und die Wirkungsweise des erfindungsgemäßen Wundverbandes, der Wundauflage oder der Trägermatrix als neuartiges System ist in den anliegenden Figuren 1 und 2 schematisch dargestellt.

Figur 1 zeigt das Grundprinzip eines erfindungsgemäßen Wundverbandes, einer Wundauflage oder einer Trägermatrix.

Figur 2 zeigt eine bevorzugte Ausführungsform dieses Prinzips im vergrößerten Maßstab.

Die Herstellung des Wundverbandes kann beispielsweise dadurch erfolgen, daß ein Gemisch aus 3 %-iger Lösung von Guar gum in PBS (phosphate buffer saline) (0,5 Mol pH 7,5) und 0,1 molarer Boraxlösung in demineralisiertem Wasser hergestellt wird. Die Mischung wird unverzüglich auf eine Schicht eines offenporigen Polyurethanschaums aufgetragen, dessen Porengröße durchschnittlich 1 bis 2 mm beträgt. Nach ca. 30 Minuten wird das ausgebildete Hydrogel mehrfach mit Wasser gewaschen.

Zumindest die der Wunde zugewandte Seite wird mit einem bifunktionellen Kopplungsreagenz, wie Glutaraldehyd, einem Diisocyanat oder Bromcyan umgesetzt und danach in üblicher Weise mit einem proteolytischen Enzym oder dem gewünschten, die Heilung fördernden Peptid. Niedermolekulare, nicht gebundene Bestandteile werden durch Auswaschen mit Wasser entfernt. Die der Wunde abgewandte Seite wird mit einer Polyetherurethanfolie abgedeckt und der Wundverband aufgerollt. Bei Bedarf werden geeignete Stücke abgeschnitten, auf die Wunde aufgelegt und mit Hilfe eines Verbandes oder einem selbstklebenden Pflaster am Körper befestigt.

Gut bewährt hat sich auch folgende Arbeitsweise zur Kopplung von Peptiden an das Hydrogel:
Das Polysaccharid-Gel wird nacheinander mit Wasser, DioxanWasser (3:7), Dioxan-Wasser (7:3) und Dioxan (20 ml jeweils für 3 g Feuchtgel-Kuchen) versetzt und abschließend in wasserfreiem Dioxan suspendiert. Statt des wasserfreien Dioxans kann auch wasserfreies Azeton genommen werden.

1,1-Carbonyldiimidazol (CDI) (120 mg für 3 g Feuchtgel) wird zugegeben und bei Raumtemperatur vorsichtig ca. 15 Minuten geschüttelt. Das ergibt ein aktiviertes Gel mit 40 bis 50 µMol aktive Gruppen pro 1 ml Feuchtgel.

Das aktivierte Gel wird mit frischem wasserfreien Dioxan oder Azeton gewaschen, um das freie Imidazol zu entfernen.

Das CDI-aktivierte Hydrogel (ca. 400 µMol/g Gel) wird mit einem handelsüblichen, gereinigten Trypsin (TPCK-behandelt; TPCK = Tosylphenylalaninchlormethan-Kaliumsalz) in 10 mM Na-borat-500 mM NaCl, pH 8,0-Puffer, 20 %-ig an Glycerol (10 mg Enzym pro ml aktiviertes Gel) bei 4°C 48 Stunden inkubiert. Dann wird mit 100 mM KH₂PO₄ - 500 mM NaCl, pH 8,0, 50 %-ig an Glycerol- 0,05 % NaN₃ gewaschen.

Die spezifische Aktivität des immobilisierten Trypsins beträgt ca. 3500 Units/ml Gel.

Weitere Herstellungsmethoden sind in dem folgenden Beispiel beschrieben:

25 g Guar gum Pulver werden in 987 ml warmer 0,2 % Agaroselösung mit Hilfe eines Dispersers vermischt. Die 0,2 %ige Agarose-Lösung wird in der Weise hergestellt, daß zunächst 2 g Agarose in 100 ml demineralisiertem Wasser gelöst wird. Dazu wird die Suspension von Agarose in Wasser bis zum Kochen erhitzt.

Die restlichen 887 ml demineralisiertes Wasser werden in einem Wasserbad auf 45 °C erwärmt. Sie enthalten bereits 0,5 % NaCl. Zu dieser erwärmten, 0,5 %igen NaCl-Lösung wird die klare Agaroselösung unter Rühren zugegeben.

In die nun vorliegende, erwärmte 0,2 %ige Agaroselösung werden 25 g Guar gum Pulver mittels eines Dispersers eingemischt. Innerhalb von wenigen Sekunden hat sich ein Gel von breiiger Konsistenz gebildet. Dieses Hydrogel wird sofort auf eine 10 cm breite, 2 - 5 mm dicke, vorgefertigte, offenporige Polyurethanschaumbahn (PUR-Schaum) aufgetragen.

Das gleichmäßige Auftragen des Guar gum-Gels auf den PUR-Schaum wird durch eine besondere Auftrageeinrichtung erreicht. Das 10 cm breite PUR-Schaumband liegt in einer nach beiden Seiten offenen Form, deren Seitenwände ca. 5 cm hoch sind, und die eine Länge von 100 cm hat. Die gleichmäßige Schichtdicke wird durch zusätzlich an beiden Seiten langlaufende Distanzstreifen gewährleistet.

Zwischen diesen Distanzstreifen liegt eingepaßt das PUR-Schaumband. Mit einem Rakel wird das auf den PUR-Schaum aufgetragene Gel aufgestrichen, indem der Rakel auf dem Distanzstreifen laufend, längs der Form entlanggezogen wird.

Durch dieses Aufstreichen des Gels mit noch breiiger Konsistenz entsteht eine innige Verbindung mit dem PUR-Schaum. Im nachfolgenden Schritt wird eine 0,1 M Boratlösung (pH 8,0) auf die Oberfläche des Gels aufgesprüht. Zum Aufsprühen können alle im Labor gebräuchlichen Zerstäuber verwendet werden. Pro 10 cm² Fläche ist eine Menge von ca. 2 ml Boratlösung ausreichend, um eine Vernetzung des Guar gum-Gels zu erreichen.

Innerhalb von ca. 10 Minuten hat sich das auf dem PUR-Schaum aufgetragene Gel von der Oberfläche nach innen fortschreitend so verfestigt, daß es nun eine stabile Wundauflage darstellt. Ein Spülvorgang mit physiologischer Kochsalzlösung entfernt die nicht gebundenen Borat-Ionen.

Die Immobilisierung von biologisch aktiven Proteinen/Peptiden an die Oberfläche des Guar gum-Hydrogels wird durch die bifunktionelle Kopplungssubstanz 1,1-Carbonyldiimidazol (CDI) erreicht.

In einem Ansatz werden 20 g Guar gum in 100 ml trockenem Aceton (über Molekularsieb; 0,4 nm) aufgeschwemmt. Die Guar gum-Suspension wird in eine Glasfritte (G 3) überführt und das Aceton mit einem leichten Vakuum (Wasserstrahlpumpe) abgesaugt. Das erhaltene Guar gum wird erneut mit 100 ml getrocknetem Aceton aufgeschlämmt, einige Minuten geschüttelt und das Aceton wieder abgesaugt.

Zum trockenen Guar gum-Pulver werden 3 g CDI, gelöst in 80 ml trockenem Aceton, zugegeben. Die Aktivierung des Guar gums erfolgt über 30 Minuten unter vorsichtigem Schütteln bei Raumtemperatur. Das Aceton mit dem nicht umgesetzten CDI wird über eine Glasfritte unter leichtem Vakuum abgesaugt. Das aktivierte Guar gum wird 5 mal mit 60 ml trockenem Aceton gewaschen, das Aceton jedesmal abgesaugt und schließlich bis zu kleinen Restmengen an Aceton abgesaugt. Das aktivierte Guar gum wird für die Immobilisierung des gewünschten Enzyms wie folgt eingesetzt:

Zur Herstellung der Wundauflage mit den immobilisierten Enzymen an der Oberfläche wird zunächst wie oben beschrieben vorgegangen. Der Unterschied dazu besteht darin, daß anstelle des Guar gum-Pulvers aktiviertes Guar gum verwendet wird.

Nach dem Aufstreichen des ebenfalls breiigen Gels mit Hilfe der oben beschriebenen Auftragungstechnologie auf den PUR-Schaum wird nun eine Lösung des zu koppelnden Enzyms in einem Boratpuffer aufgesprüht.

Für eine Hydrogelfläche von 10 cm² werden ca. 2 ml 0,1 M Boratpuffer, pH 8,0, 10 mg Collagenase enthaltend, aufgesprüht.

Die Reaktionszeit des aufgetragenenen Enzyms mit den aktiven Gruppen auf der Oberfläche des Hydrogels beträgt 1 Stunde bei Raumtemperatur und ca. 20 Stunden bei 4° C. Das modifizierte Gel wird dann mehrmals (mindestens 3x) mit physiologischer Kochsalzlösung gespült und leicht getrocknet. Die Trocknung der Hydrogele erfolgt in einem Trockenschrank bei 40° C über 24 Stunden. Die getrockneten Wundauflagen werden in Beutel aus beschichteter Aluminiumfolie eingeschweißt und mittels gamma-Sterilisation sterilisiert.

## Patentansprüche

1. Wundverband, Wundauflage oder Trägermatrix bestehend aus einem bioverträglichen, offenporigen Kunststoffschaum, in dessen Poren eingelagert ist ein Hydrogel, welches gebildet ist aus einem mit Borat modifiziertem Guar gum, an welches zumindest oberflächlich gebunden sind über freie Hydroxyl- und/oder Aminogruppen und bifunktionelle Kopplungsreagenzien, die Wundheilung fördernde Peptide und/oder eingelagert sind bakterizide oder antimykotische Substanzen.

2. Wundverband, Wundauflage oder Trägermatrix gemäß Anspruch 1, dadurch gekennzeichnet, daß die der Wunde abgewendete Seite abgedeckt ist mit einer wasserundurchlässigen, aber gas- und wasserdampfdurchlässigen Schicht.

3. Wundverband, Wundauflage oder Trägermatrix gemäß Anspruch 1 oder 2, dadurch gekennzeichnet, daß nur die der Wunde zugewendete Seite des Kunststoffschaums mit dem Hydrogel gefüllt ist, während die der Wunde abgewendete Seite noch nur mit Luft gefüllte Poren aufweist.

4. Wundverband, Wundauflage oder Trägermatrix gemäß einem der Ansprüche 1 bis 3, dadurch gekennzeichnet, daß die die Wundheilung fördernden Peptide proteolytische Enzyme sind.

5. Wundverband, Wundauflage oder Trägermatrix gemäß einem der Ansprüche 2 bis 4, dadurch gekennzeichnet, daß die Schicht selbstklebend ist.

6. Wundverband, Wundauflage oder Trägermatrix gemäß einem der Ansprüche 1 bis 5, dadurch gekennzeichnet, daß in das Hydrogel eingelagert sind weitere Wirkstoffe und/oder die Wundheilung fördernde Peptide.

7. Wundverband, Wundauflage oder Trägermatrix gemäß einem der Ansprüche 1 bis 6, dadurch gekennzeichnet, daß der Kunststoffschaum eine vorgefertigte Polyurethanschaumbahn ist.

8. Wundverband, Wundauflage oder Trägermatrix gemäß einem der Ansprüche 1 bis 7, dadurch gekennzeichnet, daß auf der Oberfläche über Zelladhäsionsmoleküle gebundene autolog gezüchtete Zellverbände vorhanden sind.

9. Wundverband, Wundauflage oder Trägermatrix gemäß Anspruch 8, dadurch gekennzeichnet, daß die Zellen gentechnologisch verändert sind.

## Claims

1. A wound dressing, wound overlay or carrier matrix, consisting of a biocompatible open-cell foam of a synthetic material, in the pores of which a hydrogel has been included, said hydrogel having been formed of a borate-modified guar gum, to which peptides promoting wound healing have been bonded at least superficially through free hydroxyl and/or amino groups and bifunctional coupling reagents, and/or in which bactericidal or antimycotic substances have been included.

2. The wound dressing, wound overlay or carrier matrix according to claim 1, characterized in that the surface remote from the wound of the dressing is covered with a layer which is water-impermeable, while permeable to gas and water vapor.

3. The wound dressing, wound overlay or carrier matrix according to claims 1 or 2, characterized in that only the surface facing the wound of the synthetic foam has been filled with the hydrogel, whereas the surface remote from the wound only comprises air-filled pores.

4. The wound dressing, wound overlay or carrier matrix according to any of claims 1 to 3, characterized in that the peptides promoting wound healing are proteolytic enzymes.

5. The wound dressing, wound overlay or carrier matrix according to any of claims 2 to 4, characterized in that the layer is self-adhesive.

6. The wound dressing, wound overlay or carrier matrix according to any of claims 1 to 5, characterized in that further active ingredients and/or peptides promoting wound healing have been incorporated in the hydrogel.

7. The wound dressing, wound overlay or carrier matrix according to any of claims 1 to 6, characterized in that the synthetic foam is a pre-fabricated polyurethane foam sheet.

8. The wound dressing, wound overlay or carrier matrix according to any of claims 1 to 7, characterized in that cell colonies which have been autologously cultivated are present on the surface bonded through cell adhesion molecules.

9. The wound dressing, wound overlay or carrier matrix according to claim 8, characterized in that the cells have been changed by genetic engineering.

## Revendications

1. Pansement, recouvrement de plaie ou matrice portante, constitué d'une mousse plastique biocompatible, à alvéoles ouverts, dans les pores de laquelle est logé un hydrogel, lequel est obtenu à partir d'une gomme de guar modifiée par un borate, gomme à laquelle, au moins en surface, sont liés des peptides favorisant la cicatrisation par l'intermédiaire de groupes hydroxyle et/ou amino libres et de réactifs de couplage bifonctionnels, et/ou dans laquelle sont incorporées des substances bactéricides ou antimycotiques.

2. Pansement, recouvrement de plaie ou matrice portante selon la revendication 1, caractérisé en ce qu'il est recouvert, sur le côté opposé à la plaie, d'une couche imperméable à l'eau, mais perméable aux gaz et à la vapeur d'eau.

3. Pansement, recouvrement de plaie ou matrice portante selon la revendication 1 ou 2, caractérisé en ce que seul le côté de la mousse plastique dirigé vers la plaie est rempli de l'hydrogel, tandis que le côté opposé à la plaie ne présente encore que des pores remplis d'air.

4. Pansement, recouvrement de plaie ou matrice portante selon l'une des revendications 1 à 3, caractérisé en ce que les peptides favorisant la cicatrisation sont des enzymes protéolytiques.

5. Pansement, recouvrement de plaie ou matrice portante selon l'une des revendications 2 à 4, caractérisé en ce que la couche est auto-adhésive.

6. Pansement, recouvrement de plaie ou matrice portante selon l'une des revendications 1 à 5, caractérisé en ce que d'autres principes actifs et/ou les peptides favorisant la cicatrisation sont incorporés dans l'hydrogel.

7. Pansement, recouvrement de plaie ou matrice portante selon l'une des revendications 1 à 6, caractérisé en ce que la mousse plastique est une feuille de mousse de polyurethanne préfabriquée.

8. Pansement, recouvrement de plaie ou matrice portante selon l'une des revendications 1 à 7, caractérisé en ce que l'on a, sur la surface, des groupements cellulaires à incubation autologue et liés par l'intermédiaire de molécules d'adhérence cellulaire.

9. Pansement, recouvrement de plaie ou matrice portante selon la revendication 8, caractérisé en ce que les cellules ont subi une modification de génie génétique.
